# EUROPEAN PATENT SPECIFICATION

(11) **EP 1 618 081 B1**
(45) Date of publication and mention of the grant of the patent: **02.01.2008**
(21) Application number: 04707818.3
(22) Date of filing: 03.02.2004
(51) Int. Cl.: C07C 2/64, C07C 15/107

(54) **METHOD OF PREPARING BRANCHED ALKYL AROMATIC HYDROCARBONS USING COMBINED PROCESS STREAMS FROM A DIMERIZATION UNIT AND AN ISOMERIZATION UNIT**
VERFAHREN ZUR HERSTELLUNG VERZWEIGTER ALKYLAROMATISCHER KOHLENWASSERSTOFFE UNTER VERWENDUNG KOMBINIERTER VERFAHRENSSTRÖME AUS EINER DIMERISATIONSEINHEIT UND EINER ISOMERISATIONSEINHEIT
PROCEDE DE PREPARATION D'HYDROCARBURES AROMATIQUES D'ALKYLE RAMIFIE AU MOYEN DE FLUX DE TRAITEMENT COMBINES DERIVES D'UNE UNITE DE DIMERISATION ET D'UNE UNITE D'ISOMERISATION

(30) Priority: 05.02.2003 US 445277 P
(43) Date of publication of application: 25.01.2006
(73) Proprietor: SHELL INTERNATIONALE RESEARCH MAATSCHAPPIJ B.V., 2596 HR Den Haag (NL)
(72) Inventor: AYOUB, Paul, Marie, NL-1031 CM Amsterdam (NL); DIRKZWAGER, Hendrik, NL-1031 CM Amsterdam (NL); MURRAY, Brendan, Dermot, Houston, TX 77077 (US); SUMROW, Steven, Clois, Katy, TX 77450 (US)
(86) International application number: PCT/US2004/003166
(87) International publication number: WO 2004/072001

(56) References cited:
- WO-A-02/44114
- US-A- 3 492 364

## Description

### 1. Field of Invention

The present invention generally relates to systems and methods for preparing alkyl aromatic hydrocarbons. More particularly, embodiments described herein relate to systems and methods for preparing branched alkyl aromatic hydrocarbons.

### 2. Description of Related Art

Alkylated aromatic hydrocarbons are important compounds that may be used in a variety of applications or converted to other chemical compounds (e.g. surfactants, sulfonates). Surfactants may be used in a variety of applications, such as detergents, soaps and oil recovery.

The structural composition of an alkyl aromatic hydrocarbon may influence the properties (*e.g*., water solubility, biodegradability, cold water detergency) of the surfactant and/or detergent produced from an alkyl aromatic hydrocarbon. For example, water solubility may be affected by linearity of the alkyl group. As the linearity of the alkyl group increases, the hydrophilicity (i.e., affinity for water) of the alkyl aromatic surfactant may decrease. Thus, the water solubility and/or detergency (performance) of the alkyl aromatic surfactant may decrease. Incorporating branches that contain a minimum number of quaternary and/or tertiary carbon atoms into the alkyl portion of the alkyl aromatic surfactant may increase the cold-water solubility and/or detergency of the alkyl aromatic surfactant while maintaining the biodegradability of a detergent The amount and type of branching of the alkyl group, however, may decrease the biodegradation rate of a surfactant and/or detergent.

Branched alkyl aromatic hydrocarbons are composed of a branched alkyl group coupled to an aromatic group. Branched alkyl groups are composed of a linear alkyl groups with branches extending form the linear alkyl group. Branches of the linear alkyl groups may include one or more aliphatic alkyl groups, a linear alkyl group or combinations thereof. Branches may include methyl, ethyl, propyl or higher alkyl groups. Quaternary and tertiary carbons may be present in a branched the alkyl group. The number of quaternary and tertiary carbons may result from the branching pattern in the alkyl group. As used herein, the phrase "aliphatic quaternary carbon atom" refers to a carbon atom that is not bound to any hydrogen atoms and not attached to an aromatic ring system.

A surfactant with a branched alkyl group including quaternary and/or tertiary carbons may have a lower biodegradation rate than a surfactant with a linear or mono-branched alkyl group. As used herein, "biodegradable" refers to a material that can be chemically altered or broken down by bacteria or other natural agents. For example, in a biodegradation experiment using a porous pot activated sludge treatment, a biodegradation rate of sodium 2-methyl-2-undecyl[¹⁴C]benzensulfonate was greater than a biodegradation rate of sodium 5-methyl-5-undecyl[¹⁴C]benzensulfonate. A detailed description of the experiment is described by Nielsen et al. in "Biodegradation of Coproducts of Commercial Linear Alkylbenzene Sulfonate", Environmental Science and Technology, 1997, 31:3397-3404.

Examples of compositions and process for the manufacture of branched alkyl aromatic hydrocarbons are described in U.S. Patent No. 3,484,498 to Berg, entitled "Process For The Preparation Of Aryl-Substituted Normal Paraffin Hydrocarbons", U.S. Patent No. 5,196,624 to Threlkel et al., entitled "Detergent Grade to C₁₀ to C₂₈ Olefins, (C₁₀ to C₂₈ Alkyl)Benzenes and C₁₀ to C₂₈ Alkyl)Benzene Sulfonates and Process for Preparing Same Using A Phosphite Containing Catalyst"; U.S. Patent No. 5,196,625 to Threlkel et al. entitled "Detergent Grade to C₁₀ to C₂₈ Olefins, (C₁₀ to C₂₈ Alkyl) Benzenes and C₁₀ to C₂₈ Alkyl) Benzene Sulfonates and Process for Preparing Same Using A Phosphite Containing Catalyst"; U.S. Patent No. 6,111,158 to Marinangeli et aL, entitled "Process For Producing Arylalkanes At Alkylation Conditions Using A Zeolite Having a NES Zeolite Structure Type"; U.S. Patent No. 6,187,981 to Marinangeli et al., entitled "Process For Producing Arylalkanes And Arylalkane Sulfonates, Compositions Produced Therefrom, and Uses Thereof," and WO-A-02/44 114.

More recently, it has been found that surfactants derived from branched olefins have different, more desirable properties than surfactants derived from linear olefins. For example, surfactants derived from branched olefins have increased water solubility, improved detergency properties and acceptable biodegradable properties compared to surfactants derived from linear olefins. Production of branched olefins from a Fischer-Tropsch process stream may increase an economic value of the stream. In some embodiments, linear olefins may be converted into branched olefins using a dimerization and/or isomerization catalyst. The branched olefins may be used to produce surfactants that are more desirable and thus more valuable to the producer of the Fischer-Tropsch stream. In general, Fischer-Tropsch processes tend to produce minor amounts of olefins. Increasing an olefin content (e.g. branched alkyl olefin content) of a Fischer-Tropsch stream may increase the economic value of the stream.

### SUMMARY

In an embodiment, an alkyl aromatic hydrocarbon may be produced by a method that includes producing olefins in a dimerization unit. The produced dimerized olefins may include branched dimerized olefins. At least a portion of the produced dimerized olefins may be used to alkylate aromatic hydrocarbons.

Process conditions in the dimerization unit may be such that the resulting branched olefins have an average number of branches per olefin molecule of between about 0.7 and about 2.5. The branched olefin may include, but is not limited to methyl and/or ethyl branching. The dimerization process may produce branched olefins that include less than about 0.5 percent of quaternary carbon atoms. In an embodiment, a feed stream entering the dimerization unit includes alpha-olefins with an average carbon number from 4 to 8. Branched olefins produced from the dimerization of alpha-olefins with an average carbon number from 4 to 8 will have an average carbon number from 8 to 16. As used herein the phrase "carbon number" refers to the total number of carbons in a molecule.

At least a portion of the unreacted components and the produced dimerized olefins may be separated to produce an unreacted hydrocarbons stream and a produced dimerized olefins stream. At least a portion of the unreacted hydrocarbons stream may be recycled to the dimerization unit.

Isomerization of olefins in a process stream may occur in an isomerization unit Isomerized olefins may be used to alkylate aromatic hydrocarbons. In an embodiment, a process feed stream entering an isomerization unit may include linear olefins and paraffins having an average carbon number from 7 to 16. In an embodiment, a process feed stream entering an isomerization unit includes linear olefins and paraffins having an average carbon number from 10 to 13. In certain embodiments, a process feed stream entering an isomerization unit is derived from a Fischer-Tropsch process. In the isomerization unit, at least a portion of the linear olefins in a hydrocarbon stream may be isomerized to branched olefins. Branched olefins may have an average number of branches per olefin molecule of between about. 0.7 and about 2.5. Branched olefins may include, but is not limited to, methyl and/or ethyl branching. The isomerization process may produce branched olefins that include less than about 0.5 percent of aliphatic quaternary carbon atoms.

In an embodiment, one or more hydrocarbon streams may be combined with the feed stream entering the isomerization unit. The hydrocarbon stream may be mixed with the feed stream to alter the concentration of the olefins entering the isomerization unit. After the feed stream is processed in the isomerization unit, the resulting branched olefin containing stream is passed into an alkylation unit One or more hydrocarbon streams may be combined with the branched olefin containing stream to alter the concentration of olefins entering the alkylation unit.

In an embodiment, at least a portion of the product stream exiting the dimerization unit may be combined with at least a portion of the product stream exiting an isomerization unit to form a combined stream. The combined stream may be transported to an alkylation unit The alkylation unit alkylates aromatic hydrocarbons with at least a portion of the olefins in the combined stream. After alkylation of the aromatic hydrocarbons, at least a portion of unreacted components from the alkylation process may be separated from the alkyl aromatic hydrocarbon products.

Alkylation of aromatic hydrocarbons with olefins may occur in an alkylation unit In an embodiment, an olefinic hydrocarbons stream from an isomerization unit, a dimerization unit, and/or a combination thereof and aromatic hydrocarbons may enter an alkylation unit. In the alkylation unit, at least a portion of the aromatic hydrocarbons may be alkylated with at least a portion of the olefins in the hydrocarbons stream to produce alkyl aromatic hydrocarbons. At least a portion of the produced alkyl aromatic hydrocarbons may include a branched alkyl group. At least a portion of the unreacted components of the hydrocarbons stream, at least a portion of unreacted aromatic hydrocarbons and at least a portion of the produced alkyl aromatic hydrocarbons may form an alkylation reaction stream.

At least a portion of the paraffins, unreacted olefins, aromatic hydrocarbons and alkyl aromatic hydrocarbons from the alkylation reaction stream may be separated to produce an unreacted hydrocarbons stream and an alkyl aromatic hydrocarbons product stream. The unreacted hydrocarbons stream may be further separated, in some embodiments, to form a paraffins and unreacted olefins stream and an aromatic hydrocarbons stream. At least a portion of the unreacted aromatic hydrocarbons stream may be recycled to the alkylation unit

In certain embodiments, at least a portion of the alkyl aromatic hydrocarbon product streams may be sulfonated to form alkyl aromatic sulfonates. In some embodiments, alkyl aromatic sulfonates may include branched alkyl groups.

### BRIEF DESCRIPTION OF THE DRAWINGS

Advantages of the present invention will become apparent to those skilled in the art with the benefit of the following detailed description of embodiments and upon reference to the accompanying drawings, in which:
FIG. 1 depicts a schematic diagram of an embodiment of a system for producing branched alkyl aromatic hydrocarbons using a dimerization unit and an isomerization unit; and
FIG. 2 depicts a schematic diagram of an embodiment of a separation unit to separate produced dimerized olefins from a reaction mixture.

While the invention is susceptible to various modifications and alternative forms, specific embodiments thereof are shown by way of example in the drawing and will herein be described in detail. It should be understood that the drawings and detailed description thereto are not intended to limit the invention to the particular form disclosed, but on the contrary, the intention is to cover all modifications, equivalents, and alternatives falling within the spirit and scope of the present invention as defined by the appended claims.

### DETAII,ED DESCRIPTION OF EMBODIMENTS

Hydrocarbon products may be synthesized from synthesis gas (i.e., a mixture of hydrogen and carbon monoxide) using a Fischer-Tropsch process. Synthesis gas may be derived from coal or by reforming of natural gas. The Fischer-Tropsch process catalytically converts synthesis gas into a mixture of products that includes primarily saturated hydrocarbons, a certain amount of olefins and a minor amount of oxygen containing products. The products from a Fischer-Tropsch process may be used for the production of fuels (*e.g*., gasoline, diesel oil), lubricating oils and waxes. Fuels and other products produced by a Fischer-Tropsch process generally have low levels of sulfur, nitrogen and/or metals and contain little or no cyclic compounds (*e.g*., (aromatics, naphthalenes).

Fischer-Tropsch process streams may be used to prepare economically valuable commodity products. For example, linear olefins are commodity products that are useful for production of surfactants. Using a portion of the process stream to produce linear olefins may increase the economic value of a Fischer-Tropsch process stream.

A hydrocarbon feed stream may be obtained from a Fischer-Tropsch process or from an ethylene oligomerization process. Fischer-Tropsch catalysts and reaction conditions may be selected to provide a particular mix of products in a reaction product stream. For example, a Fischer-Tropsch catalyst and reaction conditions may be selected to increase the amount of olefins and decrease the amount of paraffins and oxygenates in the stream. Alternatively, the catalyst and reaction conditions may be selected to increase an amount of paraffins and decrease an amount of olefins and oxygenates in the stream. Catalysts and/or combinations of catalyst that favor the manufacture of desired hydrocarbon species in a Fischer-Tropsch process are generally known.

While reference is made to a Fischer-Tropsch stream, it is to be understood that any stream, made by any process, that includes olefins and saturated hydrocarbons may be a suitable feedstock for the processes disclosed herein. Many Fischer-Tropsch streams may contain from 5 percent to 99 percent olefins, the remainder being saturated hydrocarbons comprising paraffins and other compounds.

In some embodiments, feed streams containing olefins and paraffins are obtained through cracking of paraffin wax or the oligomerization of olefins. Commercial olefin products manufactured by ethylene oligomerization are marketed in the United States by Chevron Chemical Company, Shell Chemical Company (under the trademark NEODENE), and by British Petroleum. Cracking of paraffin to produce alpha-olefin and paraffin feed streams is described in U.S. Patent No. 4,579,986 to Sie, entitled "Process For The Preparation Of Hydrocarbons" and U.S. Patent Application Serial No. 10/153,955 to Ansorge et al., entitled "Process For The Preparation of linear Olefins and Use Thereof To Prepare Linear Alcohols". Specific procedures for preparing linear olefins from ethylene are described in U.S. Patent No. 3,676,523 to Mason, entitled "Alpha-Olefin Production", U.S. Patent No. 3,686,351 to Mason, entitled "Alpha-Olefin Production; "U.S. Patent No. 3,737,475 to Mason, entitled "Alpha-Olefin Production" and U.S. Patent No. 4,020,121 to Kister et al., entitled "Oligomerization Reaction System." Most of the above-mentioned processes produce alpha-olefins. Higher linear internal olefins may be commercially produced, for example, by the chlorination-dehydrochlorination of paraffins, by paraffin dehydrogenation, or by isomerization of alpha-olefins.

In an embodiment, a feed stream is processed to produce a hydrocarbon stream that includes branched olefins. Branched olefins may be used to alkylate an aromatic hydrocarbon to produce branched alkyl aromatic hydrocarbons. The feed stream may have a paraffin content range between about 50 percent by weight to about 90 percent by weight of the feed stream. In certain embodiments, a feed stream may have a paraffin content greater than about 90 percent by weight paraffins. The feed stream may also include olefins. An olefin content of the feed stream may be between about 10 percent by weight to about 50 percent by weight. In other embodiments, a feed stream may have an olefin content greater than 90 percent by weight olefins. Composition of the feed stream may include hydrocarbons having an average carbon number ranging from 4 to 30. In an embodiment, an average carbon number of the hydrocarbons in a feed stream may range from 4 to 24. In other embodiments, an average carbon number of the feed stream may range from 4 to 16. The average carbon number of the hydrocarbons may range from 7 to 16 for processes that involve an isomerization unit. In certain embodiments, an average carbon number of the hydrocarbons may range from 10 to 13 for processes that involve an isomerization unit. The average carbon number of the hydrocarbons may range from 4 to 6 for processes that use a dimerization unit. In certain embodiments, an average carbon number of the hydrocarbons ranges from 4 to 8 for processes thai use a dimerization unit A feed stream may include minor amounts of hydrocarbons having a carbon number that is higher or lower then the desired carbon number range. In some embodiments, a feed stream may be derived from distillation of a process stream that includes a broader range of carbon numbers.

In an embodiment, a feed stream for a dimerization and/or isomerization unit includes mono-olefins and/or paraffins. Mono-olefins may be of a linear or branched structure. Mono-olefins may have an alpha or internal double bond position. The feed stream may include olefins in which 50 percent or more of the olefin molecules present may be alpha-olefins of a linear (straight chain) carbon skeletal structure. In certain embodiments, at least about 70 percent of the olefins are alpha-olefins of a linear carbon skeletal structure. A hydrocarbon stream in which greater than about 70 percent of all of the olefin molecules are alpha-olefins of a linear carbon skeletal structure may be used in certain embodiments of alkylation of aromatic hydrocarbons. Such a stream may be derived from a Fischer-Tropsch process. In some embodiments, a feed stream includes olefins in which at least about 50 percent of the olefin molecules present are internal olefins.

Branched chain olefins may be used as a feed source for an aromatic alkylation reaction. An aromatic alkylation reaction uses olefins in an incoming stream to alkylate aromatic compounds. Aromatic compounds (e.g., aromatic hydrocarbons) may include benzene or substituted benzene. In an embodiment, alkyl-substituted benzene is used as a substituted benzene in the process. Alkyl-substituted benzenes may be mono- and/or poly-substituted lower alkyl benzenes. The alkyl substituent of an alkyl substituted benzene may have a carbon number ranging from 1 to 5. In an embodiment, a carbon number of the alkyl substituent may range from 1 to 2. Suitable examples of aromatic compounds include, but are not limited to, benzene, toluene, xylenes, ethylbenzene, cumene, n-propylbenzene and other mono- and poly-lower alkyl benzenes. In some embodiments, a single aromatic compound or a mixture of two or more aromatic compounds may be used as feed source. Aromatic hydrocarbons may be fed into the reactor directly or mixed in a suitable non-reactive organic solvent prior to addition to the alkylation unit.

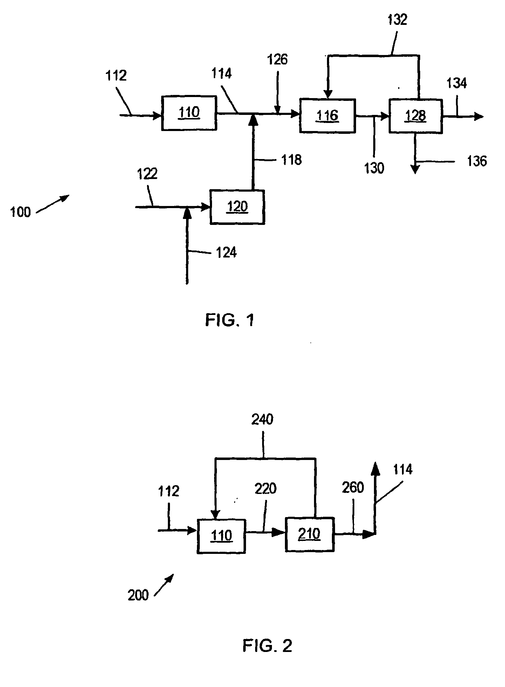

Referring to System 100 depicted in FIG. 1, a first hydrocarbon stream including olefins and paraffins may be introduced into dimerization unit 110 via first conduit 112. In certain embodiments, hydrocarbons in the first hydrocarbon stream may have an average carbon number from 4 to 8. In other embodiments, hydrocarbons in the first hydrocarbon stream may have an average carbon number from 4 to 6. The first hydrocarbon stream may be derived, in some embodiments, from a Fischer-Tropsch process. In dimerization unit 110, at least a portion of the olefins may be dimerized. At least a portion of the dimerized olefins exit dimerization unit 110 as a second hydrocarbon stream via second conduit 114. Depending on the choice of catalyst, the resulting dimer may be branched. The branches of the olefin produced in dimerization unit 110 may include methyl, ethyl and/or longer carbon chains. In an embodiment, dimerized olefins may contain greater than about 50 percent methyl branches. In some embodiments, resulting dimerized olefins may contain greater than about 90 percent methyl branches.

In certain embodiments, dimerization unit 110 may have several points of entry to accommodate process streams which may vary in composition. The process streams may be from other processing units and/or storage units. Examples of process streams include, but are not limited to, a diluent hydrocarbon stream, and/or other hydrocarbon streams that include olefins and paraffins derived from other processes. As used herein "entry into the dimerization unit" refers to entry of process streams into the isomerization unit through one or more entry points.

The dimerization catalyst used in dimerization unit 110 may be a homogeneous or heterogeneous catalyst. In certain embodiments, a dimerization catalyst used in dimerization unit 110 may be a catalyst that includes oxides of Group III, Group IVA, Group IVB, Group VIIIA. or combinations thereof. Examples of such oxides include, but are not limited to, nickel oxide, silicon dioxide, titanium dioxide, aluminum oxide or zirconium dioxide. The catalyst may include an amorphous nickel oxide (NiO) present as a dispersed substantial monolayer on the surfaces of a silica (SiO₂) support. A surface of silica support may also include minor amounts of an oxide of aluminum, gallium or indium such that the ratio of nickel oxide to metal oxide present in the catalyst is within the range of from about 4:1 to about 100:1. The dimerization catalyst may be prepared by precipitating a water insoluble nickel salt onto the surface of a silica support that has been impregnated with the metal oxide. Alternatively, a dimerization catalyst may be prepared by precipitating a water insoluble nickel salt onto a silica-alumina support, which has been dealuminized such that the resulting nickel oxide/alumina ratio falls within the range of from about 4:1 to about 100:1. The catalyst may be activated by calcination in the presence of oxygen at a temperature within a range of from about 300 °C to about 700 °C or, in some embodiments, a temperature range from about 500 °C to about 600 °C.

A silica support surface area may be within the range of from about 100 m²/g to about 450 m²/g. In an embodiment, a silica surface area may be within the range from about 200 m²/g to about 400 m²/g. A nickel oxide content dispersed in the silica support may range from about 7 percent to about 70 percent by weight. In certain embodiments, a nickel oxide content may be from about 20 percent to about 50 percent by weight, depending on the surface area of the particular support utilized in preparing a catalyst. For a silica support having a surface area of about 300 m²/g, a nickel oxide content may, in some embodiments, ranges from about 21 percent to about 35 percent by weight. A nickel oxide content may, in other embodiments, be about 28 percent by weight.

The silica support may be in dry granular form or in a hydrogel form prior to precipitation of the nickel oxide precursor compound on the surfaces thereof. Silica hydrogel may be prepared by mixing a water-soluble silicate (e.g., a sodium or potassium silicate) with a mineral acid then washed with water to remove water-soluble ions. The resulting silica hydrogel may be partially or completely dried.

A nickel oxide precursor may include a water-insoluble nickel salt, such as nickel carbonate, nickel phosphate, nickel nitrate or nickel hydroxide. The water-insoluble nickel salt may be generated in-situ by forming an aqueous mixture of silica gel and a water-soluble nickel salt. The nickel salt may include, but is not limited to, nickel nitrate, nickel sulfonate, nickel carbonylate, nickel halide. A base may be added to the aqueous mixture to induce precipitation of a water-insoluble nickel salt. The water-insoluble nickel salt, which may be formed, may be precipitated in finely divided form within the interstices and on the surface of the silica support. The treated silica support may then be recovered, washed several times and dried.

A second component in the dimerization catalyst may be a trivalent metal oxide. The trivalent metal oxides may include, but are not limited to, aluminum, gallium and indium or combinations thereof. Although, a nickel oxide and/or silica catalyst described above may be active for olefin dimerization, deactivation may occur quickly. Deactivation may be a consequence of a formation of large oligomers that remain attached to the surface and act as coke precursors. The presence of a small amount of the trivalent metal oxide within the catalyst yields acid sites. Acid sites may promote catalytic activity without promoting unwanted and/or excessive oligomer formation.

The trivalent metal oxide may be incorporated into the silica support by any suitable technique (e.g., the precipitation method described above or by direct impregnation in the form of a water-soluble salt). In an embodiment, a trivalent metal oxide may be impregnated into the silica support as an aqueous solution by the addition of a water-soluble salt The water-soluble metal salt may include, but is not limited to, metal nitrates, metal chlorides or metal sulfates. Once impregnated with a metal salt, the silica support may be dried and calcinated to reduce the metal salt to an oxide form. The resulting silica support activated with the trivalent oxide may then be further treated to incorporate the nickel oxide layer onto the silica-trivalent metal oxide support.

In some embodiment, a silica-trivalent metal oxide support may include silica/alumina, silica/gallia or silica/india gel. In certain embodiments, content of a metal oxide (e.g., alumina) present in the support may be low in comparison with the content of nickel oxide. Therefore, dealuminization of the silica/alumina gel of relatively high alumina content (e.g., above about 5 percent by weight) may be necessary to reduce the content of alumina. Dealuminization may be accomplished by techniques known in the art (e.g., extraction of the aluminum with an organic or inorganic acid). Organic or inorganic acids may include, but are not limited to, nitric acid, sulfuric acid, hydrochloric acid, chloroacetic acid or ethylene diamine tetraacetic acid. Extraction may be accomplished by adding an acid to an aqueous dispersion of the alumino silicate followed by stirring, decantation and washing with water. The process may be repeated one or more times until a desired alumina content is achieved. The solids may then be dried, calcined and further treated as described above to incorporate the nickel oxide layer onto the silica/alumina support.

The content of trivalent metal oxide with respect to the content of the nickel oxide present in the silica support may be significant, in some embodiments, in order to achieve improved results in terms of dimer yield and minimum average methyl branching present in the dimer product. In certain embodiments, when the content of trivalent metal oxide is too low, e.g., above a nickel oxide to trivalent metal oxide ratio of about 100 to 1, then the yield of dimer decreases and the catalyst may tend to deactivate quickly. In certain embodiments, when the content of trivalent metal oxide is too high, (e.g., below a nickel oxide to trivalent metal oxide ratio of about 4 to I) then the yield of dimer may tend to decrease and the average content of methyl branching in the dimer product may tend to increase. In certain embodiments, content of a trivalent metal oxide may be such that the ratio of nickel oxide to trivalent metal oxide falls within the range of from about 4:1 to about 30:1. In other embodiments, content of a trivalent metal oxide may be such that the ratio of nickel oxide to trivalent metal oxide is between about 5:1 and about 20:1. In certain embodiments, a ratio of nickel oxide to trivalent metal oxide may be between about 8:1 and about 15:1.

In certain embodiments, a dimerization catalyst may contain from about 21 percent to about 35 percent by weight of nickel oxide and about 1 percent to about 5 percent by weight of trivalent metal oxide, based on the total weight of nickel oxide, trivalent metal oxide and silica. In certain embodiments, a dimerization catalyst may include from about 1.5 percent to about 4 percent by weight trivalent metal oxide based on the total weight of nickel oxide, trivalent metal oxide and silica.

The preparation of dimerization catalysts are described in U.S. Patent 5,849, 972 to Vicari et al., entitled "Oligomerization Of Olefins To Highly Linear Oligomers, and Catalyst For This Purpose", and U.S. Patent, 5,169,824 to Saleh et al., entitled "Catalyst Comprising Amorphous NiO On Silica/Alumina Support."

Conversion of olefins in a first hydrocarbon feed stream to dimers in dimerization unit 110, may be carried out as a batch, continuous (e.g. using a fixed bed), semi-batch or multi-step process. In a batch process, the dimerization catalyst may be slurried with the first hydrocarbon feed stream. Temperature for the dimerization reaction may range from about 120 °C to about 200 °C. In an embodiment, reaction temperatures may range from about 150 °C to about 165 °C. Reaction temperatures may be controlled with evaporative cooling, e.g., the evaporation of lighter hydrocarbon fractions from the reaction mixture controls the reaction temperature.

Branched olefins produced in dimerization unit 110 may include methyl, ethyl and/or longer carbon chain branches. Hydrogen Nuclear Magnetic Resonance (¹H NMR) analysis of the isomerized olefin composition may be performed. Branched olefins may include quaternary and/or tertiary aliphatic carbons. In certain embodiments, an amount of quaternary and/or tertiary aliphatic carbons produced in a dimerization unit may be minimized. ¹H NMR analysis of the olefins produced by the dimerization unit may indicate the extent of isomerization of the olefins in the hydrocarbon stream.

The presence of quaternary carbon atoms may be determined using carbon 13 (¹³C) NMR techniques. The type of branching (e.g., methyl, ethyl, propyl or larger groups) may be determined by hydrogenation of the olefin mixture and then ¹³C NMR analysis of the hydrogenated olefin solution.

In an embodiment, an average number of branches per olefin molecule present in the branched olefin composition is between about 0.1 and about 2.5. In other embodiments, an average number of branches per olefin molecule present in the branched olefin composition is between about 0.7 and about 2.5. In certain embodiments, when the feed stream contains greater than 70 percent linear olefins, an average number of branches per olefin molecule present in the branched olefin composition is between about 0.2 and about 1.5. The degree of branching in the product may be controlled by controlling process conditions used in the dimerization unit. For example, high reaction temperatures and lower feed rates may result in a higher degree of branching. Methyl branches may represent between about 20 percent to about 99 percent of the total number of branches present in the olefin molecules. In some embodiments, methyl branches may represent greater than about 50 percent of the total number of branches in the olefin molecules. The number of ethyl branches in the olefin molecules may represent, in certain embodiments, less than about 30 percent of the total number of branches. In other embodiments, a number of ethyl branches, if present, may be between about 0.1 percent and about 2 percent of the total number of branches. Branches other than methyl or ethyl, if present, may be less than about 10 percent of the total number of branches.

The number of aliphatic quaternary and/or tertiary carbon atoms present in the branched olefin composition may be less than about 2 percent of the carbon atoms present. In an embodiment, an aliphatic quaternary and/or tertiary carbons present are less than about 1 percent of the carbon atoms present For applications in which biodegradability is important, the number of aliphatic quaternary carbon atoms may be less than about 0.5 percent of the carbon atoms present. In an embodiment, a number of aliphatic quaternary and/or tertiary carbon atoms is less than about 0.3 percent of the carbon atoms present. In other embodiments, a number of aliphatic quaternary carbon atoms present in the branched olefin composition is between about 0.01 percent and about 0.3 percent of the aliphatic carbon atoms present.

Produced dimerized olefins may exit the dimerization unit in a second hydrocarbon stream via second conduit 114. The second hydrocarbon stream may be transported to other processing units (e.g., an alkylation unit, hydroformylation unit, etc.). In some embodiments, the second hydrocarbon stream may include olefins with an average carbon number from 8 to 16. In other embodiments, the second hydrocarbon stream may include olefins with an average carbon number from 8 to 12. The second hydrocarbon stream may include, in some embodiments, an olefin content of greater than 50 percent by weight.

Produced dimerized olefins may be separated, if desired, from the reaction mixture through techniques known in the art (e.g., fractional distillation). In an embodiment, at least a portion of second hydrocarbon stream may exit dimerization unit 110 and enter separation unit 210 via conduit 220 as depicted in for System 200 in FIG. 2B. In separation unit 210 the reaction mixture may be separated into a produced dimerized olefins stream and a paraffins and unreacted olefins stream through fractional distillation. As used herein "fractional distillation" is the distillation of liquids and subsequent collection of fractions of liquids determined by boiling point. The paraffins and unreacted olefins stream may contain hydrocarbons with a carbon number less than 8. At least a portion of the paraffins and unreacted olefins stream may be introduced into dimerization unit 110 via conduit 240. The produced dimerized olefins stream may exit separation unit 210 and be introduced into second conduit 114 via conduit 260.

At least a portion of the produced dimerized olefins stream may exit dimerization unit 110 and enter alkylation unit 116 via second conduit 114. A fourth hydrocarbon stream may be introduced directly into alkylation unit 116 through one or more alkylation unit ports. At least a portion of a fourth hydrocarbon stream may be introduced into second conduit 114 via fourth conduit 118 upstream of alkylation unit 116 to produce a combined stream. The fourth hydrocarbon stream may be a stream exiting from isomerization unit 120. In certain embodiments, hydrocarbons in the fourth hydrocarbon stream may have an average carbon number range from 8 to 16 or, in some embodiments, 10 to 13.

Isomerization unit 120 may be fed by a third hydrocarbon stream containing paraffins and unreacted olefins via a third conduit 122. In isomerization unit 120, at least a portion of the olefins in the third hydrocarbon stream may be isomerized to branched olefins to produce the fourth hydrocarbon stream. The third hydrocarbon stream may include hydrocarbons with an average carbon number from 7 to 16 or, in some embodiments from 10 to 13. In some embodiments, the third hydrocarbon stream includes alpha-olefins. In certain embodiments, a third hydrocarbon stream is a stream derived from a Fischer-Tropsch process. The alpha-olefin content of the third hydrocarbon stream may be greater than about 70 percent of the total amount of olefins in the third hydrocarbon stream. In isomerization unit 120, at least a portion of the olefins in the third hydrocarbon stream may be isomerized to branched olefins (e.g., isoolefins) to produce a fourth hydrocarbon stream. ¹H NMR and ¹³C NMR analysis of the olefins produced by the isomerization unit may indicate the extent of isomerization of the olefins and the quantity of quaternary carbons in the hydrocarbon stream.

In certain embodiments, isomerization unit 120 may have several points of entry to accommodate process streams which may vary in composition. Process streams may be from other processing units and/or storage units. Examples of process streams include, but are not limited to, a diluent hydrocarbon stream, other hydrocarbon streams that include olefins and paraffins derived from other processes. As used herein "entry into the isomerization unit" is entry of process streams into the isomerization unit through one or more entry points.

Conditions for olefin isomerization in isomerization unit 120 may be controlled such that the number of carbon atoms in the olefins before and after isomerization is substantially the same. U. S. Patent No. 5,648,584 to Murray, entitled "Process for Isomerizing Linear Olefins to Isoolefins" and U.S. Patent No. 5,648,585 to Murray et al., entitled "Process for Isomerizing Linear Olefins to Isoolefins" describe catalysts and process conditions to skelctally isomerize linear olefins to branched olefins.

In an embodiment, linear olefins in a third hydrocarbon stream are isomerized in isomerization unit 120 by contacting at least a portion of the third hydrocarbon stream with a zeolite catalyst. The zeolite catalyst may have at least one channel with a crystallographic free channel diameter ranging from greater than 4.2 Å and less than about 7 Å. The zeolite catalyst may have an elliptical pore size large enough to permit entry of a linear olefin and diffusion, at least partially, of a branched olefin. The pore size of the zeolite catalyst may also be small enough to retard coke formation. As used herein, "coke" refers to the product from thermal degradation of a molecule into smaller molecules.

Temperatures at which the olefin isomerization may be conducted range from about 200 °C to about 500 °C. Temperatures in isomerization unit 120 are, in some embodiments, kept below the temperature at which the olefin will crack extensively. As used herein "cracking" is the process of thermally degrading molecules into smaller molecules. To inhibit cracking, low temperatures may be used at low feed rates. In certain embodiments, lower temperatures may be used when the amount of oxygenates present in the process stream is low. Higher feed rates may be desirable to increase the production rate of isomerized products. Higher feed rates may be used, in some embodiments, when operating at higher reaction temperatures. The reaction temperature, however, should be set such that cracking to lower boiling weight products is minimized. For example, greater than 90 percent of linear olefins may be converted to branched olefins at 230 °C at a feed rate of 60 grams per hour with minimal cracking. Pressures maintained in isomerization unit 120 may be at a hydrocarbon partial pressure ranging from about 0.1 atmosphere (10 kPa) to about 20 atmospheres (2026 kPa). In an embodiment, partial pressure may range from above about 0.5 atmosphere (51 kPa) to about 10 atmospheres (1013 kPa).

In an embodiment, at least a portion of a paraffinic hydrocarbon stream may be introduced into third conduit 122 via fifth conduit 124 upstream of isomerization unit 120 to produce a combined stream. The combined stream may enter isomerization unit 120 via third conduit 122. In other embodiments, a paraffinic hydrocarbon stream is introduced directly into isomerization unit 120 through one or more points of entry.

At least a portion of the olefins in the combined stream may be isomerized to branched olefins in isomerization unit 120 to produce a fourth hydrocarbon stream. The addition of the paraffinic hydrocarbon stream may be used to optimize the olefin concentration in isomerization unit 120 and to control the extent of branching in the produced olefins. The concentration of paraffins in the paraffinic hydrocarbon stream may be between about 10 percent and about 99 percent by weight. In certain embodiments, a paraffin concentration may range between about 10 percent and about 50 percent by weight. In some embodiments, a paraffin concentration may range between about 25 percent and about 75 percent by weight.

Alkylation of aromatic hydrocarbons by at least a portion of branched olefins produced in the stream from dimerization unit 110 and/or isomerization unit 120 may be conducted using various types of reactors. In certain embodiments, an isomerization and/or dimerization process may be carried out in a batch wise fashion by adding the catalyst and aromatic hydrocarbons to a reactor, heating the mixture to a reaction temperature, and then adding the olefinic or aromatic hydrocarbons to the heated mixture.

Alkylation unit 116 may have several points of entry to accommodate the entry of additional process streams. As used herein, "stream entering into the alkylation unit" refers to as the entry of process streams into the alkylation unit through one or more entry points. Examples of such process streams include, but are not limited to, additional streams from dimerization unit 110 and/or isomerization unit 120, a diluent hydrocarbon stream, gases and/or other hydrocarbon streams that include olefins and paraffins derived from other processes.

In an embodiment, at least a portion of the olefins in the combined hydrocarbon stream, produced by dimerization unit 110 and isomerization unit 120 is contacted with aromatic hydrocarbons (e.g., benzene) under a variety of alkylating conditions. At least a portion of the resulting alkyl aromatic hydrocarbons are monoalkylated aromatic hydrocarbons having a branched alkyl group. Minimization of other alkylation products, such as dialkylation and higher alkylation products may be controlled by the process conditions (e.g., molar ratio, reaction temperatures, catalyst type and reactant concentrations) in alkylation unit 116.

The stoichiometry of the alkylation reaction requires only one mole of aromatic hydrocarbon compound per mole of total mono-olefins. Using 1:1 molar conditions, however, tends to produce mixtures of olefin oligomers and/or polymers, monoalkyl aromatic hydrocarbons, dialkyl-, trialkyl- and possibly highly polyalkylated aromatic hydrocarbons or unreacted olefins. It is desired, however, to have the molar ratio as close to 1:1 as possible to maximize utilization of the aromatic hydrocarbon and to minimize recycle of unreacted aromatic hydrocarbons or unreacted olefins. The molar proportion of aromatic hydrocarbon to total mono-olefin may influence both conversion and selectivity of the alkylation reaction. In certain embodiments, a molar ratio of total aromatic hydrocarbon per mole of total mono-olefins may be set between about 3:1 and about 100:1 to maximize formation of monoalkyl aromatic hydrocarbon. In some embodiments, an aromatic hydrocarbon to olefin ratio may be from about 5:1 to about 50:1. In other embodiments, an aromatic hydrocarbon to olefin ratio may be from about 5:1 to about 35:1. In certain embodiments, an aromatic hydrocarbon to total olefin ratio may be from about 8:1 to about 30:1.

In an embodiment, alkylation conditions for alkylation of aromatic hydrocarbons in alkylation unit 116 may include the use of a Friedel-Crafts catalyst type. The Friedel-Crafts catalyst may be an acidic inorganic material. Examples of acidic inorganic materials include, but are not limited to, mineral acids such as sulfuric acid containing less than about 10 percent water, hydrofluoric acid containing less than about 10 percent water, liquefied anhydrous hydrogen fluoride, and/or other inorganic materials used in combination with hydrofluoric acid. Other inorganic materials may include, but are not limited to, Lewis acids such as anhydrous aluminum chloride, anhydrous aluminum bromide and boron trifluoride.

In certain embodiments, an alkylation catalyst may be a molecular sieve such as ITQ-21 in the acidic form. Synthesis and structures of molecular sieve catalysts are described by Conna, et al. in "A large-cavity zeolite with wide pore windows and potential as an oil refining catalyst," Nature, 2002, 418: 514.

In some embodiments, a catalyst used in alkylation unit 116 is based on a zeolite catalyst that may be modified with a metal or metal compound. In other embodiments, a catalyst used in alkylation unit 116 is based on a zeolite catalyst that may not be modified with a metal or metal compound. Zeolite alkylation catalysts arc described in U.S. Patent Application Serial No. 10/075,318 entitled "A Process For Preparing (Branched-Alkyl) Arylsulfonates and (Branched-Alkyl) Arylsulfonate Composition," U.S. Patent No. 6,111,158 to Marinangeli et al. entitled "Process For Producing Arylalkanes At Alkylation Conditions Using A Zeolite Having a NES Zeolite Structure Type" and U.S. Patent No. 5,041,402 to Schoennagel et al., entitled "Thermally Stable Noble Metal-Containing Zeolite Catalyst." A zeolite catalyst may have at least one channel with a crystallographic free channel diameter ranging from greater than about 4 Å to less than about 9 Å. With the understanding that when the pores have an elliptical shape, the larger pore size dimension is the dimension to be considered. In an embodiment, a pore size dimensions may range between about 5.5 Å to about 7 Å. Pore size dimensions of zeolites are described by W. M. Meier et al., "Atlas of Zeolite Structure Types," Fourth revised edition, 1996, Elsevier.

In alkylation unit 116, at least a portion of the olefins in the second hydrocarbon stream, at least a portion of the olefins in the fourth hydrocarbon stream, or combinations thereof and at least a portion of the aromatic hydrocarbons may be reacted under alkylation conditions in the presence of the alkylation catalyst Reaction temperatures for the alkylation reaction may range between greater than about 30 °C and less than about 300 °C. In certain embodiments, reaction temperatures may range between greater than about 100 °C and less than about 250 °C. An alkylation reaction may be conducted in at least a partial liquid phase, in an all-liquid phase or at supercritical conditions. In certain embodiments, pressures in the alkylation unit are sufficient to maintain reactants in the liquid phase. The pressure used in alkylation unit 116 may depend upon the identity of olefin, the identity of the aromatic hydrocarbon and/or the temperature of the reaction. Pressures in alkylation unit 116 may range between about 3 atmospheres and about 70 atmospheres (304 kPa-7095 kPa). In certain embodiments, pressures may range between about 20 atmospheres and about 35 atmospheres (2025-3545 kPa).

Alkylation conditions in alkylation unit 116 may be maintained to minimize skeletal isomerization of the branched olefins. Alkylation conditions may also be maintained to produce alkyl aromatic hydrocarbons with an alkyl group that corresponds to the branching of the olefins produced in dimerization unit 110 and/or isomerization unit 120. "Skeletal isomerization during alkylation," as used herein, refers to an isomerization that occurs during alkylation that changes the position of the branching in the olefin or alkyl aromatic hydrocarbon product. Accordingly, alkylation conditions may be set such that the number of quaternary aliphatic carbon atoms in the olefin and the alkyl aromatic hydrocarbon product may remain unchanged during the alkylation reaction.

A general class of branched alkyl aromatic compounds produced in alkylation unit 116 may be characterized by a chemical formula R-Y, in which Y represents an aromatic hydrocarbyl radical (e.g., a phenyl radical) and R represents a radical derived from an olefin produced in dimerization unit 110 and/or isomerization unit 120. The olefin may have a carbon number in the range from 7 to 16. In certain embodiments, an olefin carbon number may range from 10 to 16. The olefin carbon number may, in other embodiments, range from 10 to 13. R may be a branched alkyl radical. Branches on a branched alkyl radical may include, but arc not limited to, methyl, ethyl and/or longer carbon chains. The average number of branches per alkyl molecule present in the alkyl composition may be equal to the number of branches in the olefin produced in dimerization unit 110 and isomerization unit 120 (e.g., between 0.1 and 2.0).

In an embodiment, a fifth hydrocarbon stream may be introduced into alkylation unit 116 through one or more alkylation ports. In certain embodiments, at least a portion of a fifth hydrocarbon stream may be introduced into second conduit 114 upstream of alkylation unit 116 via sixth conduit 126 to produce a combined stream. The combined stream may enter alkylation unit 116 and at least a portion of the olefins in the combined stream may alkylate aromatic hydrocarbons to produce alkyl aromatic hydrocarbons. The resulting alkylation product may be branched alkyl aromatic hydrocarbons.

The fourth and/or fifth hydrocarbon stream may be used to regulate the olefin concentration in alkylation unit 116 at a concentration sufficient to maximize monoalkylation of the aromatic hydrocarbon and the amount of alkyl branching in the product. The fifth hydrocarbon stream may be, but is not limited to, a hydrocarbons stream containing olefin, paraffins and/or hydrocarbon solvents. In an embodiment, a paraffin content of the fifth hydrocarbon stream may be between greater than about 50 percent and less than about 99 percent relative to the total hydrocarbon content. In certain embodiments, a paraffin content of the fifth hydrocarbon stream may be between 60 and 90 percent relative to the total hydrocarbon content. In another embodiment, a paraffin content may be greater than about 80 percent relative to the total hydrocarbon content.

A combined stream, which may include, but is not limited to, a second hydrocarbon stream, a fourth hydrocarbon stream, a fifth hydrocarbon stream and/or combinations thereof, may be introduced into alkylation unit, 116 via second conduit 114. An advantage of combining the streams may be that overall production of alkyl aromatic hydrocarbons may be increased with fewer throughputs.

In an embodiment, an olefin content of a fifth hydrocarbon stream ranges between about 1 percent and about 99 percent relative to the total hydrocarbon content In certain embodiments, an olefin content of a fifth hydrocarbon stream may be between about 5 percent and about 15 percent relative to the total hydrocarbon content. In other embodiments, an olefin content of a fifth hydrocarbon stream may be greater than 80 percent relative to the total hydrocarbon stream.

In some embodiments, the fifth hydrocarbon stream may include linear olefins. The addition of a stream that includes linear olefins downstream from the isomerization and dimerization units allows the creation of an alkylation feed stream that includes a mixture of linear and branched olefins. By introducing a stream including branched and linear olefins into alkylation unit 116, a mixture of branched and linear alkyl aromatic products may be obtained. Varying the amount of linear olefins added to the alkylation feed stream may control the ratio of linear to branched alkyl aromatic products. A mixture of branched and linear alkyl aromatic hydrocarbons may have improved overall properties when converted to alkyl aromatic surfactants. Examples of improved properties include, but are not limited to, low skin and eye irritation, foaming properties, biodegradability, cold-water solubility and cold-water detergency. Applications for these surfactants include, but are not limited to, personal care products, household and industrial laundry products, hand dishwashing products, machine lubricant additives and lubricating oil formulations.

The alkylation reaction mixture stream may enter separator 128 via seventh conduit 130. In separator 128 at least two streams, an unreacted hydrocarbons stream and an alkyl aromatic hydrocarbons stream may be produced. The unreacted hydrocarbons stream may be separated into an aromatic hydrocarbons stream and a paraffins and unreacted olefins stream using techniques known in the art (e.g., distillation, solid/liquid separation, absorption, solvent extraction, etc.). The separated aromatic hydrocarbons stream may exit separator 128 and be recycled to alkylation unit 116 via eighth conduit 132. The alkyl aromatic hydrocarbons product stream may exit separator 128 and be transported via ninth conduit 134 to be stored on site, sold commercially, transported off-site, and/or utilized in other processing units. At least a portion of the paraffins and unreacted olefins stream may exit separator 128 and be combined with other process streams, sent to other processing units and/or be stored on site via tenth conduit 136. In certain embodiments, the paraffins and unreacted olefins stream may he further separated into a hydrocarbons stream including paraffins and unreacted olefins with a carbon number less than 8. The hydrocarbon stream including paraffins and unreacted olefins with a carbon number less than 8 may be introduced upstream of and/or into dimerization unit 110. In some embodiments, a hydrocarbon stream including paraffins and unreacted olefins with a carbon number greater than 8 may be introduced upstream of and/or isomerization unit 120.

At least a portion of the produced alkyl aromatic hydrocarbons may be sulfonated in a sulfonation unit to form alkyl aromatic sulfonates. In certain embodiments, alkyl aromatic hydrocarbons may contain branched alkyl groups. Sulfonation of at least a portion of the aromatic hydrocarbons in the alkyl aromatic hydrocarbons product stream may be performed by any method of sulfonation known in the art. Examples of such methods include sulfonation using sulfuric acid, chlorosulfonic acid, oleum or sulfur trioxide. Details of a sulfonation method involving an air/sulfur trioxide mixture are described in U.S. Patent No. 3,427,342 to Brooks et al., entitled "Continuous Sulfonation Process." In an embodiment, a sulfur trioxide to alkyl aromatic product molar ratio used for sulfonation is 1.03.

After the sulfonation reaction is complete, the sulfonation reaction mixture may be aged for about thirty minutes and then hydrolyzed with approximately 1% water. The resulting acid mixture may be neutralized with a base to produce a salt of the branched alkyl aromatic hydrocarbon sulfonate. Suitable neutralization bases may be hydroxides of alkali metals and alkaline earth metals, and ammonium hydroxides, which provide the cation M of the salts as described herein.

The general class of branched alkyl aromatic hydrocarbon sulfonates may be characterized by the chemical formula (R-A'-SO₃)*ₙ*M. R may represent a radical derived from the branched olefins, having an average carbon number in the range from 4 to 16. In an embodiment, an average carbon number ranges from 7 to 16. In another embodiment, an average carbon number ranges from 10 to 13. A' may represent a divalent aromatic hydrocarbyl radical, (e.g. a phenyl radical). M may be a cation selected from an alkali metal ion, an alkaline earth metal ion, an ammonium ion, and/or mixtures thereof and *n* may be a number depending on the valence of the cation(s) M, such that the total electrical charge of the complex is zero. In an embodiment, M may be sodium, magnesium or potassium ions. Magnesium and potassium ions may promote water solubility and performance of the alkyl aromatic hydrocarbon sulfonate. Examples of ammonium ions may include, but are not limited to, monoethanol amine, diethanol amine and triethanol amine. In certain embodiments, an ammonium ion may be represented by NH₄⁺. The resulting alkyl aromatic hydrocarbon sulfonate salt may be stored and/or sold as a solution (e.g. 30% aqueous solution), slurry (e.g., 60% aqueous slurry) and/or flakes (e.g., 90% dried flakes).

The branched alkyl aromatic sulfonates produced in the above-described processes may be used in a wide variety of applications. An example of an application includes detergent formulations. Detergent formulations include, but are not limited to, granular laundry detergent formulation, liquid laundry detergent formulations, liquid dishwashing detergent formulations and miscellaneous formulations. Examples of miscellaneous formulations include, but are not limited to, general purpose cleaning agents, liquid soaps, shampoos and liquid scouring agents.

### Examples

**Example 1: Isomerization of Olefins in a Fischer-Tropsch derived Hydrocarbon Stream:** Carbon monoxide and hydrogen were reacted under Fischer-Tropsch process conditions to yield a hydrocarbon mixture of linear paraffins, linear olefins, a minor amount of diencs and a minor amount of oxygenates. The Fischer-Tropsch hydrocarbon stream was separated into different hydrocarbon streams using fractional distillation techniques. A hydrocarbon stream containing olefins and paraffins with an average number of carbon atoms from 8 to 10 was obtained. The composition of the resulting C₈-C₁₀ hydrocarbon stream, tabulated in Table 1, was analysed by gas chromatography.

**Table 1**

| **Fischer-Tropsch Hydrocarbon Stream Composition** | **Wt%** |
|---|---|
| C₇ and lighter hydrocarbons | 0.12 |
| C₈ branched olefins | 0.02 |
| C₈ linear olefins | 0.75 |
| l-Octene | 0.69 |
| n-Octane | 2.21 |
| C₉ branched olefins | 0.16 |
| C₉ linear olefins | 8.52 |
| l-Nonene | 8.07 |
| n-Nonane | 20.03 |
| C₁₀ branched olefins | 0.28 |
| C₁₀ linear olefins | 22.92 |
| l-Decene | 20.87 |
| n-Decane | 41.12 |
| C₁₁ and heavier hydrocarbons | 0.21 |
| C₉-C₁₁ alcohols | 3.56 |

A zeolite catalyst used for isomerization of linear olefins in the hydrocarbon stream was prepared in the following manner. Ammonium-ferrierite (645 grams) exhibiting a 5.4% loss on ignition and exhibiting the following properties: molar silica to alumina ratio of 62:1, surface area of 369 square meters per gram (P/Po = 0.03), soda content of 480 ppm and n-hexane sorption capacity of 7.3 g per 100 g of ammonium-ferrierite was loaded into a Lancaster mix muller. CATAPAL® D alumina (91 grams) exhibiting a loss on ignition of 25.7% was added to the muller. During a five-minute mulling period, 152 milliliters of deionized water was added to the alumina/ammonium-ferrierite mixture. Next, a mixture of 6.8 grams glacial acetic acid, 7.0 grams of citric acid and 152 milliliters of deionized water was slowly added to the alumina/ammonium-ferrierite mixture in the muller to peptize the alumina. The resulting alumina/ammonium-ferrierite/acid mixture was mulled for 10 minutes. Over a period of 15 minutes, a mixture of 0.20 grams of tetraamine palladium nitrate in 153 grams of deionized water was slowly added to mulled alumina/ammonium-ferrierite/acid mixture. The resulting mixture exhibited a 90:10 ratio of zeolite to alumina and a loss on ignition of 43.5%. The zeolite/alumina mixture was shaped by extruding the mixture through a stainless steel die plate (1/16" holes) of a 2.25 inch Bonnot extruder.

The moist zeolite/alumina extrudate was dried at 125 °C for 16 hours. After drying, the zeolite/alumina extrudate was longsbroken manually. The zeolite/alumina extrudate was calcined in flowing air at 200 °C for two hours. The temperature was raised to a maximum temperature of 500 °C and the zeolite/alumina extrudate was calcined for an additional two hours to yield an isomerization catalyst. The isomerization catalyst was allowed to cool in a dessicator under a nitrogen atmosphere.

Stainless steel tubing, 1 inch OD, 0.6 inch ID and 26 inches long, was used as an isomerization reactor. A thermowell extended 20 inches from the top of the stainless steel reactor tube. To load the reactor tube, the reactor tube was inverted and a piece of glass wool was transferred down the wall of the reactor tube, over the thermowell and positioned at the bottom of the reactor tube to serve as a plug for the reactor tube. Silicon carbide (20 mesh) was added to a depth of about 6 inches to the reactor tube. A second piece of glass wool was placed over the silicon carbide. A mixture of 6.0 grams of the isomerization catalyst particles (6-20 mesh) and 45 grams of fresh silicon carbide (60-80 mesh) was added to the reactor tube in two parts. The two-part addition distributed the isomerization catalyst evenly in the reactor tube and resulted in an isomerization catalyst bed of about 10 inches in length. A third piece of glass wool was added to the top of the catalyst in the reactor tube. Silicon carbide (20 mesh) was layered onto the third piece of glass wool. A fourth piece of glass wool was positioned over the silicon carbide to serve as a plug for the bottom of the reactor tube. To monitor the temperature of the reaction at various points in the reactor tube, a multipoint thermocouple was inserted into the thermowell of the reactor tube. The temperature above, below and at three different places in the catalyst bed was monitored. The reactor tube was inverted and installed in the furnace. The reactor tube was heated to an operating temperature of 280 °C over a four-hour period under flowing nitrogen. Once the temperature of 280 °C was obtained, the reactor tube was held at the operating temperature for an additional two hours to condition the isomerization catalyst.

After conditioning the isomerization catalyst, the hydrocarbon stream was pumped through the reactor tube at a flow rate of 60 g/hr. Nitrogen, at a flow rate of 6 L/hr, was passed over the isomerization catalyst simultaneously with the hydrocarbon stream. The hydrocarbon stream was vaporized before contacting the isomerization catalyst. The reactor tube was operated at an outlet pressure of 20 kPa above atmospheric pressure.

In Table 2, the weight percent of C₈-C₁₀ branched olefins, C₈-C₁₀ linear olefins and C₈-C₁₀ paraffins in the hydrocarbon stream at 0 hours and in the reactor tube effluent after 24 and 48 hours of isomerization is tabulated. Greater than 90% of the linear olefins in the hydrocarbon stream were converted into branched olefins in the isomerization reactor. During the isomerization step, a small amount of material boiling below C₈ was generated from cracking side reactions. In addition, a portion of the C₉-C₁₁ alcohols present in the feed was dehydrated to yield additional olefins in the product. The average number of alkyl branches on the C₈-C₁₀ olefins in the product was found to be 1.0 as determined by the ¹H NMR techniques.

**Table 2**

| **Fischer-Tropsch Hydrocarbon Stream Composition During Isomerization Reaction** | **0 Hr Wt%** | **24 Hr Wt%** | **48 Hr Wt%** |
|---|---|---|---|
| C₈-C₁₀ branched olefins | 0.46 | 33.04 | 33.16 |
| C₈-C₁₀ linear olefins | 32.19 | 2.52 | 2.54 |
| C₈-C₁₀ paraffins | 63.19 | 63.32 | 63.27 |
| Branched to linear C₈-₁₀ olefin ratio | 0.1 | 13.1 | 13.1 |

**Example 2. Isomerization of 1-Dodecene:** 1-Dodecene was obtained from Shell Chemical Co. The composition of 1-dodecene, as assayed by gas chromatography, is tabulated in Table 3.

**Table 3**

| **1-Dodecene Composition** | **Wt %** |
|---|---|
| 1-Dodecene | 98.0 |
| Other C₁₀-C₁₄ olefins | 1.2 |
| <C₁₀ hydrocarbons | 0.2 |
| >C₁₄ hydrocarbons | 0.2 |
| Paraffins | 0.4 |
| Total C₁₀-C₁₄ hydrocarbons | 99.6 |

1-Dodecene was isomerized using the same reactor tube design and isomerization catalyst preparation as described in Example 1. A stream of 1-dodecene was pumped through a reactor tube at a flow rate of 90 g/hr. Nitrogen, at a flow rate of 6 L/hr, was passed over the isomerization catalyst simultaneously with the stream of 1-dodecene. The stream of 1-dodecene was vaporised before contacting the isomerization catalyst. The reactor tube was operated at an outlet pressure of 20 kPa above atmospheric pressure and a temperature of 290°C.

Table 4 is a tabulation of the weight percent of hydrocarbons with carbon numbers less than C₁₀, from C₁₀-C₁₄ and greater than C₁₄ molecules in 1-dodecene at 0 hours and the reactor tube effluent after 168 and 849 hours. Linear C₁₀-C₁₄ olefins were converted in a 94% yield to branched C₁₀-C₁₄ olefins after a 168 hr processing time. During the isomerization step less than 3 weight percent of material boiling below C₁₀ was generated from cracking side reactions. The average number of alkyl branches on the C₁₀-C₁₄ olefins in the product was determined to be 1.3 by the ¹H NMR techniques.

**Table 4**

| **1-Dodecene Stream Composition During Isomerization Reaction** | **0 Hr Wt%** | **168 Hr Wt%** | **849 Hr Wt%** |
|---|---|---|---|
| <C₁₀ hydrocarbons | 0.2 | 2.5 | 2.4 |
| C₁₀-C₁₄ hydrocarbons | 99.6 | 97.2 | 97.4 |
| >C₁₄ hydrocarbons | 0.2 | 0.3 | 0.2 |
| Branched C₁₀-C₁₄ olefins | 0.6 | 93.2 | 93.4 |
| Linear C₁₀-C₁₄ olefins | 99.0 | 2.8 | 2.9 |
| Paraffins | 1.0 | 2.0 | 1.9 |

**EXAMPLE 3 :** Dimerization of 1-Hexene: A nickel oxide dimerization catalyst for the dimerization of a C₆ olefin stream was prepared by the method described for Example 1 in U.S. Patent No. 5,169,824 to Saleh et al., entitled "Catalyst Comprising Amorphous NiO On Silica/Alumina Support" The dimerization results are tabulated in Table 5.

**Table 5**

| **Test Results.** | **Example 3** |
|---|---|
| Conversion (wt%) | 59 |
| C₁₂ olefin dimer (wt%) | 22 |
| % Conversion of branched C₁₂ olefins to total C₁₂ olefins | 77 |

**EXAMPLE 4: DIMERIZATlON OF DILUTED 1-HEXENE:** A nickel oxide dimerization catalyst for the dimerization of a C₆ olefin stream was prepared by the method described for Example 1 in U.S. Patent No. 5,169,824 to Saleh et al., entitled "Catalyst Comprising Amorphous NiO On Silica/Alumina Support." A 15 mL reactor tube of the autoclave unit was charged with the NiO catalyst (0.335 grams), 1-hexene (1.675 grams), hexane (1.675 grams) and a gas chromatography standard (0.67 grams linear tetradecane). The dimerization results are tabulated in Table 6.

**Table 6**

| **Test Results.** | **Example 4** |
|---|---|
| Conversion (wt%) | 54 |
| C₁₂ olefin dimer (wt%) | 8 |
| % Conversion of branched C₁₂olefins to total C₁₂ olefins | 82 |

## Claims

1. A method for the production of alkyl aromatic hydrocarbons, comprising:
introducing a first hydrocarbon stream comprising olefins and paraffins into a dimerization unit, wherein the dimerization unit is configured to dimerize at least a portion of the olefins in the first hydrocarbon stream to produce dimerized olefins, and wherein at least a portion of the unreacted components of the first hydrocarbon stream and the produced dimerized olefins form a second hydrocarbon stream, and wherein at least a portion of the dimerized olefins are branched olefins;
introducing at least a portion of the second hydrocarbon stream and aromatic hydrocarbons into an alkylation unit;
introducing a third hydrocarbon stream into an isomerization unit, wherein the isomerization unit is configured to isomerize at least a portion of linear olefins in the third hydrocarbon stream to branched olefins, and wherein at least a portion of the unreacted components of the third hydrocarbon stream and the produced branched olefins form a fourth hydrocarbon stream; and
introducing at least a portion of the fourth hydrocarbon stream into the alkylation unit, wherein the alkylation unit is configured to alkylate at least a portion of the aromatic hydrocarbons with at least a portion of the olefins from the second hydrocarbon stream and the fourth hydrocarbon stream to produce alkyl aromatic hydrocarbons, wherein a least a portion of the produced alkyl aromatic hydrocarbons comprise a branched alkyl group.

2. The method of claim 1, wherein the first hydrocarbon stream is produced from a Fischer-Tropsch process.

3. The method of any one of claims 1 to 2, wherein the first hydrocarbon stream comprises olefins.

4. The method of any one of claims 1 to 3, wherein the second hydrocarbon stream comprises olefins having a carbon number from 10 to 16.

5. The method of any one of claims 1 to 4, wherein a portion of the branched olefins comprise an average number of branches per total olefin molecules of at least 0.7.

6. The method of any one of claims 1 to 5, wherein a portion of the branched olefins comprise methyl and ethyl branches.

7. The method of any one of claims 1 to 6, wherein the branched groups on the branched olefins are greater than 50 percent methyl groups, or less than 10 percent ethyl groups or less than 5 percent other than methyl or ethyl groups.

8. The method of any one of claims 1 to 7, wherein the branched olefins have less than 0.3 percent aliphatic quaternary carbon atoms.

9. The method of any one of claims 1 to 8, further comprising:
separating the produced dimerized olefins from the second hydrocarbon stream to form a produced dimerized olefins stream and a paraffins and unreacted olefins stream, wherein the paraffins and unreacted olefins stream comprises hydrocarbons of carbon numbers less than 7; and
introducing at least a portion of the paraffins and unreacted olefins stream into the dimerization unit.

10. The method of any one of claims 1 to 9, further comprising adjusting a ratio of olefins to paraffins introduced into the isomerization unit by introducing at least a portion of a paraffinic hydrocarbon stream into the isomerization unit.

11. The method of any one of claims 1 to 9, further comprising:
adjusting a ratio of olefins to paraffins introduced into the isomerization unit by combining a paraffinic hydrocarbon stream with at least a portion of the first hydrocarbon stream upstream of the isomerization unit to form a combined stream; and
introducing the combined stream into the isomerization unit.

12. The method of any one of claims 1 to 11, wherein the alkylation unit is configured to produce greater than 50 percent of monoalkylated aromatic hydrocarbons.

13. The method of any one of claims 1 to 12, wherein a molar ratio of the aromatic hydrocarbons to the branched. olefins is between 0.1 and 2.0 in the alkylation unit.

14. The method of any one of claims 1 to 13, wherein the alkylation unit is operated at a reaction temperature between 30 °C and 300 °C.

15. The method of any one of claims 1 to 14, wherein the branched alkyl groups of the alkyl aromatic hydrocarbons comprise 0.5 percent or less aliphatic quaternary carbon atoms and an average number of branches per alkyl group of at least 0.7, the branches comprising methyl and ethyl branches.

16. The method of any one of claims 1 to 15, wherein introducing at least a portion of the fourth hydrocarbon stream into the alkylation unit comprises combining at least a portion of the fourth hydrocarbon stream with at least a portion of the second hydrocarbon stream to produce a combined stream upstream of the alkylation unit.

17. The method of any one of claims 1 to 16, further comprising adjusting a ratio of olefins to paraffins introduced into the alkylation unit by introducing at least a portion of a fifth hydrocarbon stream into the alkylation unit; or by introducing at least a portion of a fifth hydrocarbon stream into the alkylation unit, wherein the fifth hydrocarbon stream comprises greater than 90 percent paraffins by weight.

18. The method of any one of claims 1 to 16, further comprising:
adjusting a ratio of olefins to paraffins introduced into the alkylation unit by combining at least a portion of a fifth hydrocarbon stream with at least a portion of the second hydrocarbon stream upstream of the alkylation unit to
form a combined stream or combining at least a portion of a fifth hydrocarbon stream with at least a portion of the second hydrocarbon stream upstream of the alk-ymon unit to form a combined stream, wherein the fifth hydrocarbon stream comprises greater than 90 percent paraffins by weight; and
introducing the combined stream into the alkylation unit.

19. The method of any one of claims 1 to 18, further comprising:
forming an alkylation reaction stream wherein the alkylation reaction stream comprises at least a portion of the aromatic hydrocarbons, at least a portion of the unreacted components of the second hydrocarbon stream and at least a portion of the produced alkyl aromatic hydrocarbons;
separating alkyl aromatic hydrocarbons from the alkylation reaction stream to produce an unreacted hydrocarbons stream and an alkyl aromatic hydrocarbons stream; the unreacted hydrocarbons stream comprising at least a portion of the unreacted components of the second hydrocarbon stream and at least a portion of the aromatic hydrocarbons;
separating at least a portion of the paraffins and at least a portion of the olefins from the unreacted hydrocarbons stream to produce an aromatic hydrocarbons stream and a paraffins and unreacted olefins stream; and
introducing at least a portion of the paraffins and unreacted olefins stream into the isomerization unit.

20. The method of claim 19, further comprising separating the olefins from the paraffins and unreacted olefins stream to produce an olefinic stream, wherein the average carbon number of the olefins in the olefinic stream is from 4 to 8 and introducing at least a portion of the olefinic stream into the dimerization unit.

21. The method of any one of claims 1 to 20, further comprising introducing at least a portion of the alkyl aromatic hydrocarbons stream into a sulfonation unit, wherein the sulfonation unit is configured to sulfonate at least a portion of the alkyl aromatic hydrocarbons in the alkyl aromatic hydrocarbons stream to produce alkyl aromatic sulfonates and wherein at least a portion of the alkyl aromatic sulfonates produced comprise branched alkyl aromatic sulfonates.

## Patentansprüche

1. Verfahren zur Herstellung von alkylaromatischen Kohlenwasserstoffen, umfassend:
das Einführen eines ersten Kohlenwasserstoffstroms, welcher Olefine und Paraffine umfasst, in eine Dimerisierungseinheit, wobei die Dimerisierungseinheit konfiguriert ist, um wenigstens einen Teil der Olefine im ersten Kohlenwasserstoffstrom zu dimerisieren, um dimerisierte Olefine herzustellen, und wobei wenigstens ein Teil der nicht umgesetzten Komponenten des ersten Kohlenwasserstoffstroms und der hergestellten dimerisierten Olefine einen zweiten Kohlenwasserstoffstrom ausbilden, und wobei wenigstens ein Teil der dimerisierten Olefine verzweigte Olefine sind;
das Einführen von wenigstens einem Teil des zweiten Kohlenwasserstoffstroms und aromatischer Kohlenwasserstoffe in eine Alkylierungseinheit;
das Einführen eines dritten Kohlenwasserstoffstroms in eine Isomerisierungseinheit, wobei die Isomerisierungseinheit konfiguriert ist, um wenigstens einen Teil der linearen Olefine im dritten Kohlenwasserstoffstrom zu verzweigten Olefinen zu isomerisieren, und wobei wenigstens ein Teil der nicht umgesetzten Komponenten des dritten Kohlenwasserstoffstroms und die hergestellten verzweigten Olefine einen vierten Kohlenwasserstoffstrom ausbilden; und
das Einführen von wenigstens einem Teil des vierten Kohlenwasserstoffstroms in die Alkylierungseinheit, wobei die Alkylierungseinheit konfiguriert ist, um wenigstens einen Teil der aromatischen Kohlenwasserstoffe mit wenigstens einem Teil der Olefine aus dem zweiten Kohlenwasserstoffstrom und dem vierten Kohlenwasserstoffstrom zu alkylieren, um alkylaromatische Kohlenwasserstoffe herzustellen, wobei wenigstens ein Teil der hergestellten alkylaromatischen Kohlenwasserstoffe eine verzweigte Alkylgruppe umfasst.

2. Verfahren nach Anspruch 1, wobei der erste Kohlenwasserstoffstrom aus einem Fischer-Tropsch-Verfahren stammt.

3. Verfahren nach einem der Ansprüche 1 bis 2, wobei der erste Kohlenwasserstoffstrom Olefine umfasst.

4. Verfahren nach einem der Ansprüche 1 bis 3, wobei der zweite Kohlenwasserstoffstrom Olefine mit Kohlenstoffzahlen von 10 bis 16 umfasst.

5. Verfahren nach einem der Ansprüche 1 bis 4, wobei ein Teil der verzweigten Olefine eine durchschnittliche Zahl von Verzweigungen pro Gesamtolefinmoleküle von wenigstens 0,7.

6. Verfahren nach einem der Ansprüche 1 bis 5, wobei ein Teil der verzweigten Olefine Methyl- und Ethylverzweigungen umfasst.

7. Verfahren nach einem der Ansprüche 1 bis 6, wobei die verzweigten Gruppen an den verzweigten Olefinen mehr als 50 % Methylgruppen oder weniger als 10 % Ethylgruppen oder weniger als 5 % andere Gruppen als Methyl- oder Ethylgruppen sind.

8. Verfahren nach einem der Ansprüche 1 bis 7, wobei die verzweigten Olefine weniger als 0,3 % aliphatische quaternäre Kohlenstoffatome aufweisen.

9. Verfahren nach einem der Ansprüche 1 bis 8, ferner umfassend:
das Abtrennen der hergestellten dimerisierten Olefine aus dem zweiten Kohlenwasserstoffstrom, um einen hergestellten dimerisierten Olefinstrom und einen Strom aus Paraffinen und nicht umgesetzten Olefinen herzustellen, wobei der Strom aus Paraffinen und nicht umgesetzten Olefinen Kohlenwasserstoffe mit Kohlenstoffzahlen von weniger als 7 umfasst; und
das Einführen von wenigstens einem Teil des Stroms aus Paraffinen und nicht umgesetzten Olefinen in die Dimerisierungseinheit.

10. Verfahren nach einem der Ansprüche 1 bis 9, ferner umfassend das Einstellen eines Verhältnisses von Olefinen zu Paraffinen, welche in die Isomerisierungseinheit eingeführt werden, durch Zusetzen von wenigstens einem Teil eines paraffinischen Kohlenwasserstoffstroms in die Isomerisierungseinheit.

11. Verfahren nach einem der Ansprüche 1 bis 9, ferner umfassend:
das Einstellen eines Verhältnisses von Olefinen zu Paraffinen, welche in die Isomerisierungseinheit eingeführt werden, durch Kombinieren eines paraffinischen Kohlenwasserstoffstroms mit wenigstens einem Teil des ersten Kohlenwasserstoffstroms, stromaufwärts von der Isomerisierungseinheit, um einen kombinierten Strom auszubilden; und
das Einführen des kombinierten Stroms in die Isomerisierungseinheit.

12. Verfahren nach einem der Ansprüche 1 bis 11, wobei die Alkylierungseinheit konfiguriert ist, um mehr als 50 % an monoalkylierten aromatischen Kohlenwasserstoffen herzustellen.

13. Verfahren nach einem der Ansprüche 1 bis 12, wobei ein Molverhältnis von den aromatischen Kohlenwasserstoffen zu den verzweigten Olefinen in der Alkylierungseinheit von 0,1 bis 2,0 beträgt.

14. Verfahren nach einem der Ansprüche 1 bis 13, wobei die Alkylierungseinheit bei einer Reaktionstemperatur von 30°C bis 300°C betrieben wird.

15. Verfahren nach einem der Ansprüche 1 bis 14, wobei die verzweigten Alkylgruppen der alkylaromatischen Kohlenwasserstoffe 0,5 % oder weniger an aliphatischen quaternären Kohlenstoffatomen und eine durchschnittliche Zahl von Verzweigungen pro Alkylgruppe von wenigstens 0,7 umfassen, wobei die Verzweigungen Methyl- und Ethylverzweigungen umfassen.

16. Verfahren nach einem der Ansprüche 1 bis 15, wobei das Einführen von wenigstens einem Teil des vierten Kohlenwasserstoffstroms in die Alkylierungseinheit das Kombinieren von wenigstens einem Teil des vierten Kohlenwasserstoffstroms mit wenigstens einem Teil des zweiten Kohlenwasserstoffstroms umfasst, um einen kombinierten Strom, stromaufwärts der Alkylierungseinheit, herzustellen.

17. Verfahren nach einem der Ansprüche 1 bis 16, ferner umfassend das Einstellen eines Verhältnisses von Olefinen zu Paraffinen, welche in die Alkylierungseinheit eingeführt werden, durch Einführen von wenigstens einem Teil eines fünften Kohlenwasserstoffstroms in die Alkylierungseinheit, oder durch Einführen von wenigstens einem Teil eines fünften Kohlenwasserstoffstroms in die Alkylierungseinheit, wobei der fünfte Kohlenwasserstoffstrom mehr als 90 Gew.-% Paraffine umfasst.

18. Verfahren nach einem der Ansprüche 1 bis 16, ferner umfassend:
das Einstellen eines Verhältnisses von Olefinen zu Paraffinen, welche in die Alkylierungseinheit eingeführt werden, durch Kombinieren von wenigstens einem Teil eines fünften Kohlenwasserstoffstroms mit wenigstens einem Teil des zweiten Kohlenwasserstoffstroms, stromaufwärts der Alkylierungseinheit, um einen kombinierten Strom auszubilden, oder durch Kombinieren von wenigstens einem Teil eines fünften Kohlenwasserstoffstroms mit wenigstens einem Teil des zweiten Kohlenwasserstoffstroms, stromaufwärts der Alkylierungseinheit, um einen kombinierten Strom auszubilden, wobei der fünfte Kohlenwasserstoffstrom mehr als 90 Gew.-% Paraffine umfasst; und
das Einführen des kombinierten Stroms in die Alkylierungseinheit.

19. Verfahren nach einem der Ansprüche 1 bis 18, ferner umfassend:
das Ausbilden eines Alkylierungsreaktionsstroms, wobei der Alkylierungsreaktionsstrom wenigstens einen Teil der aromatischen Kohlenwasserstoffe, wenigstens einen Teil der nicht umgesetzten Komponenten des zweiten Kohlenwasserstoffstroms und wenigstens einen Teil der hergestellten alkylaromatischen Kohlenwasserstoffe umfasst;
das Abtrennen der alkylaromatischen Kohlenwasserstoffe aus dem Alkylierungsreaktionsstrom, um einen nicht umgesetzten Kohlenwasserstoffstrom und einen alkylaromatischen Kohlenwasserstoffstrom auszubilden, wobei der nicht umgesetzte Kohlenwasserstoffstrom wenigstens einen Teil der nicht umgesetzten Komponenten des zweiten Kohlenwasserstoffstroms und wenigstens einen Teil der
aromatischen Kohlenwasserstoffe umfasst;
das Abtrennen von wenigstens einem Teil der Paraffine und wenigstens einem Teil der Olefine aus dem nicht umgesetzten Kohlenwasserstoffstrom, um einen aromatischen Kohlenwasserstoffstrom und einen Strom aus Paraffinen und nicht umgesetzten Olefinen herzustellen; und
das Einführen von wenigstens einem Teil des Stroms aus Paraffinen und nicht umgesetzten Olefinen in die Isomerisierungseinheit.

20. Verfahren nach Anspruch 19, ferner umfassend das Abtrennen der Olefine aus dem Strom aus Paraffinen und nicht umgesetzten Olefinen, um einen olefinischen Strom herzustellen, wobei die Olefine im olefinischen Strom eine mittlere Kohlenstoffzahl von 4 und 8 aufweisen, und das Einführen von wenigstens einem Teil des olefinischen Stroms in die Dimerisierungseinheit.

21. Verfahren nach einem der Ansprüche 1 bis 20, ferner umfassend das Einführen von wenigstens einem Teil des alkylaromatischen Kohlenwasserstoffstroms in eine Sulfonierungseinheit, wobei die Sulfonierungseinheit konfiguriert ist, um wenigstens einen Teil der alkylaromatischen Kohlenwasserstoffe im alkylaromatischen Kohlenwasserstoffstrom zu sulfonieren, um alkylaromatische Sulfonate herzustellen, wobei wenigstens ein Teil der hergestellten alkylaromatischen Sulfonate verzweigte alkylaromatische Sulfonate umfasst.

## Revendications

1. Procédé pour produire des hydrocarbures alkylaromatiques, comprenant :
- l'introduction d'un premier courant d'hydrocarbures comprenant des oléfines et des paraffines dans une unité de dimérisation, l'unité de dimérisation étant configurée pour dimériser au moins une partie des oléfines dans le premier courant d'hydrocarbures dans le but de produire des oléfines dimérisées, et dans laquelle au moins une partie des composants qui n'ont pas réagi du premier courant d'hydrocarbures forme avec les oléfines dimérisées produites, un deuxième courant d'hydrocarbures, et dans laquelle au moins une partie des oléfines dimérisées sont constituée d'oléfines ramifiées ;
- l'introduction d'au moins une partie du deuxième courant d'hydrocarbures et des hydrocarbures aromatiques dans une unité d'alkylation ;
- l'introduction d'un troisième courant d'hydrocarbures dans une unité d'isomérisation, l'unité d'isomérisation étant configurée pour isomériser au moins une partie des oléfines linéaires dans le troisième courant d'hydrocarbures sous forme d'oléfines ramifiées, et dans laquelle au moins une partie des composants n'ayant pas réagi du troisième courant d'hydrocarbures forme avec les oléfines ramifiées produites, un quatrième courant d'hydrocarbures ; et
- l'introduction d'au moins une partie du quatrième courant d'hydrocarbures dans l'unité d'alkylation, l'unité d'alkylation étant configurée pour alkyler au moins une partie des hydrocarbures aromatiques avec au moins une partie des oléfines provenant du deuxième courant d'hydrocarbures et du quatrième courant d'hydrocarbures pour produire des hydrocarbures alkylaromatiques, au moins une partie des hydrocarbures alkylaromatiques comprenant un groupement alkyle ramifié.

2. Procédé selon la revendication 1, dans lequel le premier courant d'hydrocarbures est produit par un procédé de Fischer-Tropsch.

3. Procédé selon l'une quelconque des revendications 1 et 2, dans lequel le premier courant d'hydrocarbures comprend des oléfines.

4. Procédé selon l'une quelconque des revendications 1 à 3, dans lequel le deuxième courant d'hydrocarbures comprend des oléfines ayant un nombre de carbone de 10 à 16.

5. Procédé selon l'une quelconque des revendications 1 à 4, dans lequel une partie des oléfines ramifiées comprend un nombre moyen de ramifications par molécules d'oléfine totales d'au moins 0,7.

6. Procédé selon l'une quelconque des revendications 1 à 5, dans lequel une partie des oléfines ramifiées comprend des ramifications de type méthyle et de type éthyle.

7. Procédé selon l'une quelconque des revendications 1 à 6, dans lequel les groupements ramifiés sur les oléfines ramifiées sont des groupements méthyle à plus de 50 pour-cent ou des groupements éthyle à moins de 10 pour-cent ou des autres groupements différents du méthyle ou de l'éthyle à moins de 5 pour-cent.

8. Procédé selon l'une quelconque des revendications 1 à 7, dans lequel les oléfines ramifiées contiennent moins de 0,3 pour-cent d'atomes de carbone quaternaires aliphatiques.

9. Procédé selon l'une quelconque des revendications 1 à 8, comprenant également :
- la séparation des oléfines dimérisées produites du deuxième courant d'hydrocarbures pour former un courant d'oléfines dimérisées produites et un courant de paraffines et d'oléfines n'ayant pas réagi, le courant de paraffines et d'oléfines n'ayant pas réagi comprenant des hydrocarbures ayant des nombres d'atomes de carbone inférieurs à 7 ; et
- l'introduction d'au moins une partie du courant de paraffines et d'oléfines n'ayant pas réagi dans l'unité de dimérisation.

10. Procédé selon l'une quelconque des revendications 1 à 9, comprenant encore le réglage d'un rapport des oléfines aux paraffines introduites dans l'unité d'isomérisation en introduisant au moins une partie d'un courant d'hydrocarbures paraffinique dans l'unité d'isomérisation.

11. Procédé selon l'une quelconque des revendications 1 à 9, comprenant en outre :
- le réglage d'un rapport des oléfines aux paraffines introduites dans l'unité d'isomérisation en combinant un courant d'hydrocarbures paraffinique avec au moins une partie du premier courant d'hydrocarbures en amont de l'unité d'isomérisation, pour former un courant combiné ; et
- l'introduction du courant combiné dans l'unité d'isomérisation.

12. Procédé selon l'une quelconque des revendications 1 à 11, dans lequel l'unité d'alkylation est configurée pour produire une quantité supérieure à 50 pour-cent d'hydrocarbures aromatiques monoalkylés.

13. Procédé selon l'une quelconque des revendications 1 à 12, dans lequel un rapport molaire des hydrocarbures aromatiques aux oléfines ramifiées se situe dans la plage de 0,1 à 2,0 dans l'unité d'alkylation.

14. Procédé selon l'une quelconque des revendications 1 à 13, dans lequel l'unité d'alkylation est exploitée à une température réactionnelle comprise entre 30 °C et 300 °C.

15. Procédé selon l'une quelconque des revendications 1 à 14, dans lequel les groupements alkyle ramifiés des hydrocarbures alkylaromatiques comprennent 0,5 pour-cent, ou moins, d'atomes de carbone quaternaires aliphatiques et un nombre moyen de ramifications par groupement alkyle d'au moins 0,7, les ramifications comprenant des ramifications de type méthyle et de type éthyle.

16. Procédé selon l'une quelconque des revendications 1 à 15, dans lequel l'introduction d'au moins une partie du quatrième courant d'hydrocarbures dans l'unité d'alkylation comprend la combinaison d'au moins une partie du quatrième courant d'hydrocarbures avec au moins une partie du deuxième courant d'hydrocarbures, pour produire un courant combiné en amont de l'unité d'alkylation.

17. Procédé selon l'une quelconque des revendications 1 à 16, comprenant en outre le réglage d'un rapport des oléfines aux paraffines introduites dans l'unité d'alkylation en introduisant au moins une partie d'un cinquième courant d'hydrocarbures dans l'unité d'alkylation ; ou en introduisant au moins une partie d'un cinquième courant d'hydrocarbures dans l'unité d'alkylation, le cinquième courant d'hydrocarbures comprenant plus de 90 pour-cent de paraffines en poids.

18. Procédé selon l'une quelconque des revendications 1 à 16, comprenant encore :
- le réglage d'un rapport des oléfines aux paraffines introduites dans l'unité d'alkylation en combinant au moins une partie d'un cinquième courant d'hydrocarbures avec au moins une partie du deuxième courant d'hydrocarbures en amont de l'unité d'alkylation, pour former un courant combiné, ou en combinant au moins une partie d'un cinquième courant d'hydrocarbures avec au moins une partie du deuxième courant d'hydrocarbures en amont de l'unité d'alkylation pour former un courant combiné, dans lequel le cinquième courant d'hydrocarbures comprend plus de 90 pour-cent en poids de paraffines ; et
- l'introduction du courant combiné dans l'unité d'alkylation.

19. Procédé selon l'une quelconque des revendications 1 à 18, comprenant également :
- la formation d'un courant de réaction d'alkylation, le courant de réaction d'alkylation comprenant au moins une partie des hydrocarbures aromatiques, au moins une partie des composants n'ayant pas réagi du deuxième courant d'hydrocarbures et au moins une partie des hydrocarbures alkylaromatiques produits ;
- la séparation des hydrocarbures alkylaromatiques du courant de réaction d'alkylation pour produire un courant d'hydrocarbures n'ayant pas réagi et un courant d'hydrocarbures alkylaromatiques ; le courant d'hydrocarbures n'ayant pas réagi comprenant au moins une partie des composants n'ayant pas réagi du deuxième courant d'hydrocarbures et au moins une partie des hydrocarbures aromatiques ;
- la séparation d'au moins une partie des paraffines et d'au moins une partie des oléfines du courant d'hydrocarbures n'ayant pas réagi, pour produire un courant d'hydrocarbures aromatiques et un courant de paraffines et d'oléfines n'ayant pas réagi ; et
- l'introduction d'au moins une partie du courant de paraffines et d'oléfines n'ayant pas réagi dans l'unité d'isomérisation.

20. Procédé selon la revendication 19, comprenant encore la séparation des oléfines du courant de paraffines et d'oléfines n'ayant pas réagi, pour produire un courant oléfinique, le nombre moyen de carbone des oléfines du courant oléfinique se situant dans la plage de 4 à 8, et l'introduction d'au moins une partie du courant oléfinique dans l'unité de dimérisation.

21. Procédé selon l'une quelconque des revendications 1 à 20, comprenant, en outre, l'introduction d'au moins une partie du courant d'hydrocarbures alkylaromatiques dans une unité de sulfonation, l'unité de sulfonation étant configurée pour sulfoner au moins une partie des hydrocarbures alkylaromatiques dans le courant d'hydrocarbures alkylaromatiques, afin de produire des alkylaromatiquesulfonates ; et dans lequel au moins une partie des alkylaromatiquesulfonates produits comprend des alkylaromatiqueulfonates ramifiés.
